# EUROPEAN PATENT APPLICATION

(11) **EP 0 583 992 A1**
(43) Date of publication of application: **23.02.1994**
(21) Application number: 93306601.1
(22) Date of filing: 20.08.1993
(51) Int. Cl.: A61B 17/32

(54) **Shield for scalpel blade**

(30) Priority: 20.08.1992 ZA 926279; 22.02.1993 ZA 931224
(71) Applicant: Shaer, Winston Daniel, Cape Town, Cape Province (ZA)
(72) Inventor: Shaer, Winston Daniel, Cape Town, Cape Province (ZA)
(74) Representative: Laredo, Jack Joseph

(57) **Abstract**

To prevent accidental cuts being inflicted by a scalpel blade, a shield is provided which comprises a resiliently flexible stem (22) and a pair of shield members (24) which, in use, lie one on each side of the scalpel blade and conceal the blade cutting edge. The scalpel handle (12) by which the shield is carried comprises a blade mount for removably locating a scalpel blade and a guide (16) through which the stem of the shield passes. The stem is resiliently flexible. As the stem is pushed through the guide and the shield positioned so that the shield members are one on each side of the blade, the stem is deflected by the non-cutting upper edge of the blade. The distortion of the stem locks the shield in place on the handle. The shield members have curved lower edges (26) which, as the scalpel is used, contact the patient on either side of the operative site. Pressing the shield members against the patient resiliently deflects the stem, thereby displacing the shield members with respect to the blade until eventually the blade cutting edge is exposed. In another form there is a tie (36) for securing the shield to the handle.

## Description

### FIELD OF THE INVENTION

THIS INVENTION relates to scalpels and to shields for scalpel blades.

### BACKGROUND TO THE INVENTION

Whilst a surgeon is carrying out an operative procedure using a scalpel, it is obligatory that trained and certified theatre staff are present and on occasion one or more surgical assistants may be needed. In passing a scalpel or changing a blade, or even when operating, the surgeon, staff or a surgical assistant may accidently be cut by the scalpel. Other potential dangers are that a scalpel may fall from the operating table or a portion of the blade may snap-off and fly across the theatre. Such injuries hold significant potential dangers particularly now that the patient's blood may be contaminated with HIV or hepatitis viruses. Avoidance of any accidental cut has become a matter of great concern to all theatre personnel.

An unprotected scalpel blade also poses dangers after the operation as it may become entangled with other waste and inflict a cut on an unwary member of the theatre staff.

### OBJECT OF THE INVENTION

The main object of the present invention is to provide a shield for a scalpel blade which shield adopts its blade shielding position immediately the blade is lifted from the operative site.

A further object of the present invention it to provide a shield for a scalpel blade which can readily be retracted to enable the scalpel blade to be changed, and then returned to its operative position.

Yet another object of the invention is to provide a shield for a scalpel blade which shield can readily be retracted if the surgical procedure requires exposure of the blade.

### BRIEF DESCRIPTION OF THE INVENTION

According to one aspect of the present invention there is provided a shield for a scalpel blade, the shield comprising an elongated stem, a pair of shield members extending downwardly from the stem, said shield members in use lying one on either side of said blade with the stem above the blade, said stem being resiliently flexible so that in use the shield members can be displaced with respect to the blade between a first position in which the blade's cutting edge is between the shield members and a second position in which said cutting edge is exposed.

In one form said stem is attached to said shield members at the forward ends only of said shield members and stem, there being gaps between the rearward parts of the shield members and the stem. Preferably the lower edges of the shield members are of a downwardly curved convex shape. The curved peripheral edge portions of the shield members can be thicker than the adjacent parts of the shield member so that no damage is inflicted on the tissue across which the shield is drawn.

According to a further aspect of the present invention there is provided in combination a scalpel comprising a handle, a scalpel blade mount integral with the handle, and a guide integral with the handle, the guide defining a passage, and a retractable scalpel blade shield comprising an elongate, resiliently flexible mounting stem and a pair of shield members, the shield members, in use, lying one on each side of a scalpel blade secured to said mount and said stem passing through said passage of said guide to locate said shield in position with respect to the handle.

According to a still further aspect of the present invention there is provided a shield for a scalpel blade, the shield comprising a pair of parallel shield members which, in use, lie one on each side of a scalpel blade, an elongate, resiliently flexible stem integral with said shield members and protruding therefrom, a flexible band secured to said stem at a location remote from said members, a face of the band being toothed, and an element having a passage therethrough, there being at least one tooth in said passage, the arrangement being such that said band can be looped around a scalpel handle, passed through said passage and pulled tight about the handle, said tooth in the passage and the teeth on the band preventing the band being removed from the passage.

### BRIEF DESCRIPTION OF THE DRAWINGS

For a better understanding of the present invention, and to show how the same may be carried into effect, reference will now be made, by way of example, to the accompanying drawings in which:-
Figure 1 is a side elevation of a scalpel handle, a scalpel shield being shown in outline;
Figure 2 is a top plan view of the scalpel handle;
Figure 3 is a section on the line III-III of Figure 1;
Figure 4 is a section on the line IV-IV of Figure 1;
Figure 5 is a side elevation of a scalpel shield;
Figure 6 is a top plan view of a scalpel shield;
Figures 7 and 8 are sections taken respectively on the lines VII-VII and VIII-VIII of Figure 5;
Figure 9 is a side elevation illustrating a modified form of shield;
Figure 10 is a top plan view of the shield of Figure 9;
Figure 11 is an end elevation of the shield of Figures 9 and 10, Figure 11 being taken in the direction of arrow A in Figure 10; and
Figure 12 is a section on the line XII-XII of Figure 9.

### DETAILED DESCRIPTION OF THE DRAWINGS

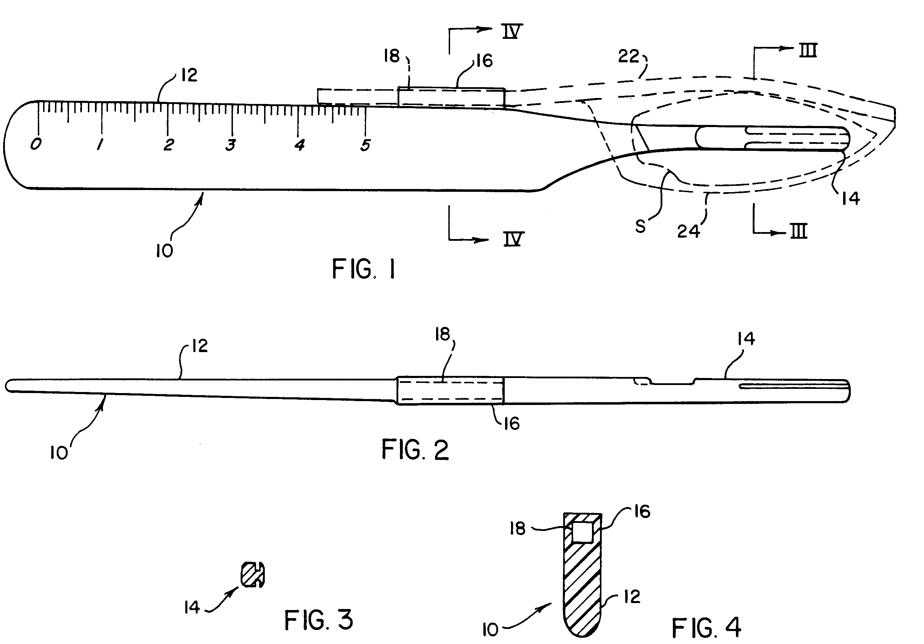

Referring firstly to Figures 1 to 4, the handle illustrated is generally designated 10 and is a one piece moulding of synthetic plastics material. The handle includes a gripping portion 12 and a blade mount 14 protruding from one end of the gripping portion 12. A guide 16 for the stem of a blade shield (which will be described in more detail hereinafter) is moulded integrally with the portion 12. The passage through the guide 16 is designated 18.

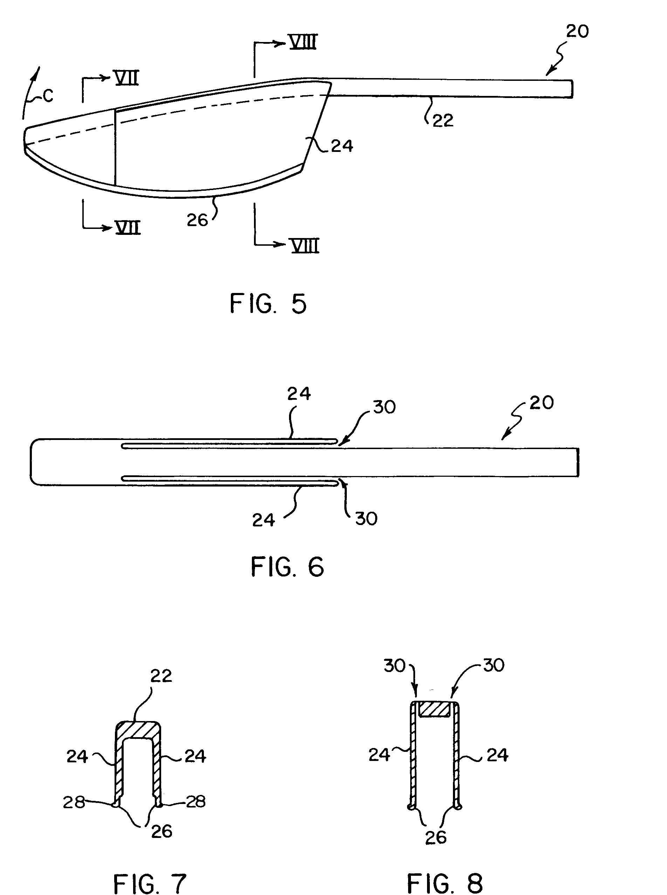

Referring now to Figures 5 to 8, the blade shield illustrated is generally designated 20 and comprises a stem 22 and two shield members 24 which protrude downwardly from the stem 22. The shield 20 is of a material which is resiliently flexible. Each member 24 has a curved lower edge 26. The members 24 are spaced apart sufficiently far in the horizontal direction to receive the blade mount 14 and a scalpel blade (not shown) between them. The curved edge 26 of each shield member 24 is thickened at 28 (see Figures 7 and 8) and of somewhat rounded form so that it does not damage the skin at the operative site as it is drawn over the skin whilst the scalpel is cutting. The stem 22 is attached to the members 24 over a part of the length of each member 24. The attached portion is at the left hand end thereof as viewed in Figures 5 and 6. The connection between the stem 22 and members 24 is best seen in Figures 6 and 7. Over the rest of the length of each member 24 there is a gap 30 (see Figures 6 and 8) between the upper edge Dortion of each member 24 and the stem 22.

To use the scalpel handle and shield, a scalpel blade s is fitted to the blade mount 14 and the stem 22 is inserted through the passage 18 of the guide 16 (see Figure 1). The scalpel blade lies between the members 24. As the shield 20 is pushed into its operative postion, the convex, non-cutting top edge of the blade s bears on the underside of the part of the stem 22 which is between the shield members 24. The stem 22 is flexed upwardly so that it is eventually slightly bent (as shown in Figure 1). The non-cutting edge of the blade s is slightly above the passage 18 to impart the desired configuration to the stem. The stem is thus fixed in position and it does not move with respect to the handle unless pushed longitudinally by the user.

The stem 22 is sufficiently resiliently flexible so that in use the forward end of the shield 20 can move upwardly, as shown by arrow C in Figure 5, with respect to the portion of the stem 22 which is fixed in position by the guide 16.

The natural flexibility of the material of the shield 20 (and which is preferably transparent crystal polycarbonate) provides the requisite flexibility. It will be understood that in the unflexed condition of the blade shield, the cutting edge of the blade is inaccessible. When the curved edges 26 of the shield members 24 are pressed against the operative site, the stem 22 flexes and the blade in effect moves down with respect to the shield members 24 until the curved cutting edge of the blade is co-incident with the curved edges 26 of the shield members 24. The requisite incision can be made by applying a little more cutting pressure. During cutting, the cutting edge of the blade is exposed below the edges 26 of the shield members 24. As soon as cutting pressure ceases, the blade is in effect retracted to its position between the members 24.

To change a blade, the entire shield 20 is slid to the left, as illustrated in Figure 1, such movement being guided by the stem 22 which slides through the guide 16. The vertical side portions of the guide 16 which are above the handle 10 slide into the gaps 30 so that the guide 16 does not interfere with retraction of the shield 20. Movement of the shield to the left is limited by contact between said vertical side portions and the end walls of the gaps 30. The blade can be removed from the blade mount 14 whilst the shield 20 is retracted and a sterile blade secured to the blade mount 14. Thereafter, the shield 20 is slid to the right and hence back to its operative position.

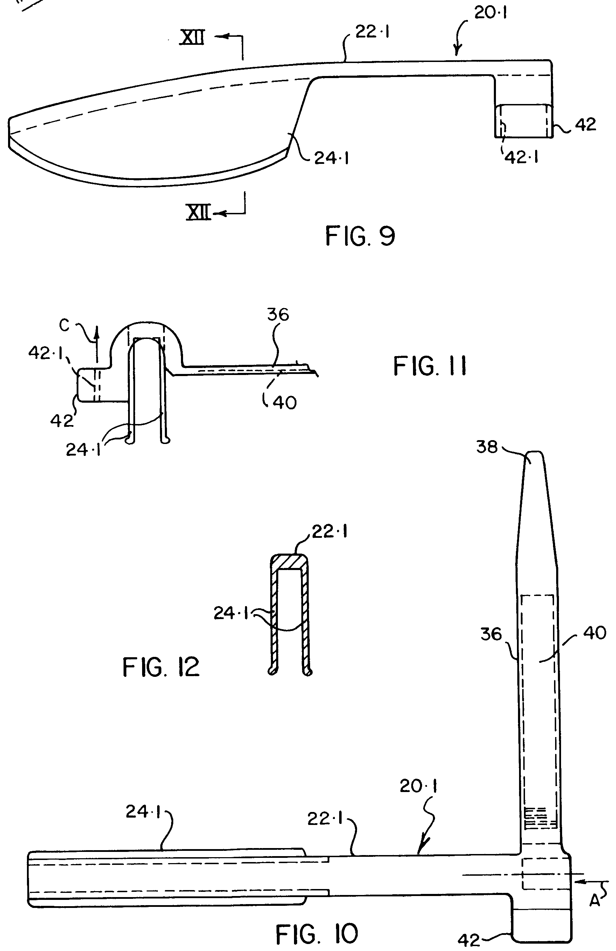

In Figures 10 to 13, the scalpel blade shield illustrated is designated 20.1, its resiliently flexible stem 22.1 and its shield members 24.1. At the end of the stem 22.1 remote from the shield members 24.1 is a flexible band 36. One end of the band 36 is moulded integrally with the stem 22.1. The other end of the band is in the form of a tapered lead-in portion 38 and there is a plurality of transversely extending teeth 40 on one face of the band 36. The shield 20.1 is intended for use with a conventional non-disposable scalpel blade handle. Such a handle will normally be of metal.

Moulded integrally with the shield 20.1 is projection 42 (see Figure 3) which has a passage 42.1 through it. In the passage 42.1 there are teeth (not shown) which co-operate with the teeth 40 of the band 36. The inter-engaging teeth in the passage 42.1 and the band prevent the band being pulled back out of the passage 42.1 once it **has** been inserted.

The shield 20.1 is intended for use with a metal non-disposable scalpel handle. To secure the blade shield 20.1 to a scalpel handle, the stem 22.1 is placed against the top of the handle with the band 36 on one side of the handle and the projection 42 on the other side. The band 36 is looped around the handle and the lead-in portion 38 of the band 36 is then pushed through the passage 42.1, as shown by the arrow C in Figure 11, so that the teeth inter-engage. The band 36 is pulled through the passage 42.1 until it fits tightly around the scalpel blade handle. The shield 20.1 can only be removed from the handle by cutting through the band 36 as the band cannot be pulled back out of the passage 42.1.

Because of the configuration of non-disposable metal handles, the stem 22.1 has to be thicker than the stem 22 so as to achieve the requisite distortion of the stem by the top edge of the blade.

## Claims

1. A shield for a scalpel blade, the shield comprising an elongate stem, a pair of shield members extending downwardly from the stem, said shield members in use lying one on either side of said blade with the stem above the blade, said stem being resiliently flexible so that in use the shield members can be displaced with respect to the blade between a first position in which the blade's cutting edge is between the shield members and a second position in which said cutting edge is exposed.

2. A shield according to claim 1, in which said stem is attached to said shield members at the forward ends only of said shield members and stem, there being gaps between the rearward parts of the shield members and the stem.

3. A shield according to claim 1, in which the lower edges of the shield members are of a downwardly curved convex shape.

4. A shield according to claim 3, in which curved peripheral edge portions of the shield members are thicker than the adjacent parts of the shield member.

5. In combination, a scalpel comprising a handle, a scalpel blade mount integral with the handle, and a guide integral with the handle, the guide defining a passage, and a retractable scalpel blade shield comprising an elongate, resiliently flexible mounting stem and a pair of shield members, the shield members, in use, lying one on each side of a scalpel blade secured to said mount and said stem passing through said passage of said guide to locate said shield in position with respect to the handle.

6. A shield for a scalpel blade, the shield comprising a pair of parallel shield members which, in use, lie one on each side of a scalpel blade, an elongate, resiliently flexible stem integral with said shield members and protruding therefrom, a flexible band secured to said stem at a location remote from said members, a face of the band being toothed, and an element having a passage therethrough, there being a least one tooth in said passage, the arrangement being such that said band can be looped around a scalpel handle, passed through said passage and pulled tight about the handle, said tooth in the passage and the teeth on the band preventing the band being removed from the passage.
